# EUROPEAN PATENT SPECIFICATION

(11) **EP 3 329 011 B1**
(45) Date of publication and mention of the grant of the patent: **18.12.2019**
(21) Application number: 16750486.9
(22) Date of filing: 28.07.2016
(51) Int. Cl.: C12Q 1/68, C12N 15/10

(54) **METHOD AND COMPOSITION**
VERFAHREN UND ZUSAMMENSETZUNG
MÉTHODE ET COMPOSITION

(30) Priority: 30.07.2015 GB 201513492; 22.01.2016 GB 201601210
(43) Date of publication of application: 06.06.2018
(73) Proprietor: Arcis Biotechnology Holdings Limited, Warrington, Cheshire WA4 4FS (GB)
(72) Inventor: ROGERS, Jan, Chester CH2 2RA (GB); REEVES, Paul, Liverpool L19 8HU (GB); TORO RUEDA, Carlos, 28022 Madrid (ES)
(74) Representative: Appleyard Lees IP LLP
(86) International application number: PCT/GB2016/052320
(87) International publication number: WO 2017/017461

(56) References cited:
- EP-A1- 1 946 761
- WO-A1-01/73110
- WO-A1-2013/024072
- WO-A1-2013/175188
- WO-A1-2014/131906
- WO-A1-2014/155078
- US-A1- 2007 190 526
- RUDY G. E. VAN EIJSDEN ET AL: "A universal fixation method based on quaternary ammonium salts (RNAlater) for omics-technologies: Saccharomyces cerevisiae as a case study", BIOTECHNOLOGY LETTERS, vol. 35, no. 6, 1 June 2013 (2013-06-01), pages 891-900, XP055307925, NL ISSN: 0141-5492, DOI: 10.1007/s10529-013-1163-0
- MEDEIROS M ET AL: "Optimization of RNA yield, purity and mRNA copy number by treatment of urine cell pellets with RNAlater", JOURNAL OF IMMUNOLOGICAL METHODS, ELSEVIER SCIENCE PUBLISHERS B.V.,AMSTERDAM, NL, vol. 279, no. 1-2, 1 August 2003 (2003-08-01), pages 135-142, XP004455313, ISSN: 0022-1759, DOI: 10.1016/S0022-1759(03)00237-0
- Daniel Gilbert Weber ET AL: "Assessment of mRnA and microRnA stabilization in peripheral Human Blood for Multicenter studies and Biobanks Biomarker Insights", Biomarker Insights, 1 January 2010 (2010-01-01), pages 95-102, XP055308017, Retrieved from the Internet: URL:https://www.ncbi.nlm.nih.gov/pmc/artic les/PMC2956623/pdf/bmi-2010-095.pdf
- TAMILVANAN S: "Oil-in-water lipid emulsions: implications for parenteral and ocular delivering systems", PROGRESS IN LIPID RESEARCH, PERGAMON PRESS, PARIS, FR, vol. 43, no. 6, 1 November 2004 (2004-11-01), pages 489-533, XP004621515, ISSN: 0163-7827, DOI: 10.1016/J.PLIPRES.2004.09.001
- PISSUWAN D ET AL: "The forthcoming applications of gold nanoparticles in drug and gene delivery systems", JOURNAL OF CONTROLLED RELEASE, ELSEVIER, AMSTERDAM, NL, vol. 149, no. 1, 5 January 2011 (2011-01-05), pages 65-71, XP027593315, ISSN: 0168-3659, DOI: 10.1016/J.JCONREL.2009.12.006 [retrieved on 2009-12-11]
- GHOSH P ET AL: "Gold nanoparticles in delivery applications", ADVANCED DRUG DELIVERY REVIEWS, ELSEVIER, AMSTERDAM, NL, vol. 60, no. 11, 17 August 2008 (2008-08-17), pages 1307-1315, XP022849514, ISSN: 0169-409X, DOI: 10.1016/J.ADDR.2008.03.016 [retrieved on 2008-04-10]
- LI GUANG-FENG ET AL: "Preparation and Testing of Quaternized Chitosan Nanoparticles as Gene Delivery Vehicles", APPLIED BIOCHEMISTRY AND BIOTECHNOLOGY, HUMANA PRESS, INC, UNITED STATES, vol. 175, no. 7, 17 February 2015 (2015-02-17), pages 3244-3257, XP035476772, ISSN: 0273-2289, DOI: 10.1007/S12010-015-1483-8 [retrieved on 2015-02-17]
- KLUMP ET AL: "Thermodynamic values of the helix-coil transition of DNA in the presence of quaternary ammonium salt", BIOCHIMICA ET BIOPHYSICA ACTA, N. NUCLEIC ACIDS AND PROTEINSYTHESIS, AMSTERDAM, NL, vol. 475, no. 4, 19 April 1977 (1977-04-19), pages 605-610, XP024554475, ISSN: 0005-2787, DOI: 10.1016/0005-2787(77)90321-5 [retrieved on 1977-04-19]
- SHARMA P ET AL: "Pure drug and polymer based nanotechnologies for the improved solubility, stability, bioavailability and targeting of anti-HIV drugs", ADVANCED DRUG DELIVERY REVIEWS, ELSEVIER, AMSTERDAM, NL, vol. 62, no. 4-5, 18 March 2010 (2010-03-18), pages 491-502, XP026917995, ISSN: 0169-409X, DOI: 10.1016/J.ADDR.2009.11.019 [retrieved on 2009-11-18]
- RESCHEL T V ET AL: "Physical properties and in vitro transfection efficiency of gene delivery vectors based on complexes of DNA with synthetic polycations", JOURNAL OF CONTROLLED RELEASE, ELSEVIER, AMSTERDAM, NL, vol. 81, no. 1-2, 17 May 2002 (2002-05-17) , pages 201-217, XP004351117, ISSN: 0168-3659, DOI: 10.1016/S0168-3659(02)00045-7

## Description

The present invention relates to methods and compositions for use in the stabilisation of genetic material such as DNA and/or RNA.

Methods of DNA and/or RNA extraction are well known in the art and have several applications across diagnostics, pharmaceuticals, and research. For example, such methods are used in the genetic engineering of plants and animals, for the diagnosis of many medical conditions, in the manufacture of a number of pharmaceuticals, and in genetic fingerprinting and crime scene investigations.

However genetic material, in particular RNA, is very fragile and often degrades very quickly. In particular it is difficult to store and transport RNA. Currently RNA must be purified prior to storage or transport. Furthermore RNA must be purified prior to its use in an amplification process such as a reverse transcription polymerase chain reaction (RT-PCR) where, typically, complementary DNA (cDNA) is produced from the RNA and a target sequence from the cDNA is subsequently amplified. Typically once RNA has been purified it is stored in nuclease-free water at -80°C or at -20°C following ethanol precipitation. However storage at such low temperatures is often not possible and is inconvenient and costly. Storage under such conditions is particularly difficult when samples are taken in less developed countries. However it would be useful to be able to use genetic material in the diagnosis of diseases that are prevalent in such countries for example malaria. Furthermore, ethanol precipitation has the disadvantage that samples must be pelleted and dissolved in buffer each time a sample is required.

WO2014/131906 discloses the use of a deep eutectic solvent to inhibit the degradation of a DNA and/ or RNA, wherein said solvent comprises a quaternary ammonium salt and silica gel. US2007/190526 describes the isolation and stabilisation of RNA from a biological sample with the use of material comprising silica and a quaternary ammonium compound. WO2013/024072 refers to the isolation and stabilisation of nucleic acids with the use of quaternary ammonium compounds and silica.

There are a number of reasons why it may be desirable to store a sample containing genetic material. Transportation of samples to an appropriate testing facility is one reason. Genetic testing may involve complex or expensive processes and thus it may be advantageous to collect and stack samples for testing in batches.

The present invention has particular utility in, for example, diagnostics and personalised medicine.

It is an aim of the present invention to provide improved means for stabilising genetic material.

According to a first aspect of the present invention there is provided a method of stabilising genetic material in a sample as defined in claim 1.

The present invention involves stabilisation of genetic material. Suitably the genetic material is selected from DNA and RNA.

In some embodiments the genetic material comprises DNA.

In some embodiments the genetic material comprises RNA.

In some embodiments the genetic material comprises DNA and RNA.

Suitable types of DNA include genomic DNA from a human, animal or microorganism, transcriptomic DNA from a human or animal, plasmid DNA, DNA aptamers, viral DNA and cosmid DNA.

Suitable types of RNA include messenger RNA (mRNA), ribosomal RNA (rRNA), signal recognition particle RNA (SRP RNA), transfer RNA (tRNA), transfer messenger RNA (tmRNA), small nuclear RNA (snRNA), small nucleolar RNA (snoRNA), SmY RNA, small Cajal body specific RNA (scaRNA), guide RNA (gRNA), ribonuclease P (RNase P), ribonuclease MRP (RNase MRP), Y RNA, telomerase RNA component (TERC), spliced leader RNA (SL RNA), antisense RNA (asRNA), cis-natural antisense RNA (cis-NAT), long noncoding RNA (IncRNA), microRNA (miRNA), piwi-interacting RNA (piRNA), small interfering RNA (siRNA), trans-acting siRNA (tasiRNA), repeat associated siRNA (rasiRNA), 7SK RNA (7SK), viral RNA and RNA aptamers. Suitably these types of RNA may be from, for example, a human, animal, or microorganism.

DNA and/or RNA from microorganisms may be from bacteria, fungi or viruses.

The sample used in the method of the present disclosure may be taken from a plant or from an animal, for example a human.

In some embodiments the sample is obtained from a plant. In such embodiments the method may be used to detect whether the plant is infected with a disease or if the plant has a genetic defect.

The sample used in the method of the present invention is preferably a sample of bodily fluid or tissue obtained from a human or other animal. Preferably the sample is a sample of bodily fluid or tissue obtained from a human. Suitable bodily fluids include blood and blood components, mucus, saliva, urine, vomit, faeces, sweat, semen, vaginal secretion, tears, pus, sputum and pleural fluid. Suitable tissues include cancer cells, connective tissue cells, epithelial cells, nervous tissue cells, muscle tissue cells and endodermal, mesodermal or ectodermal cells.

It is particularly advantageous that bodily fluid or tissue samples can be used directly in the method of the present invention. For example it is possible to carry out the method of the present invention on a whole blood sample or a sputum sample.

In some preferred embodiments the sample is a blood sample, for example a whole blood sample.

In some preferred embodiments the sample is a tissue sample, for example cancer cells.

When the sample is obtained from a plant it may comprise a portion of the leaves, roots, stem, sap, flower, seed or fruit or the plant.

The method of the present disclosure involves contacting the sample with a composition comprising (i) a quaternary ammonium compound or a precursor thereof.

Any suitable quaternary ammonium compound may be included in component (i).

Some suitable quaternary ammonium compounds have the structure (I): 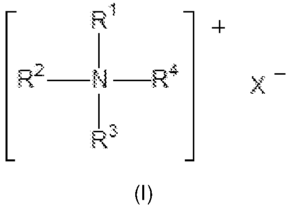 wherein each of R¹, R², R³ and R⁴ is an optionally substituted alkyl, alkenyl, alkylaryl or aryl group and X⁻ is a suitable anion. Preferably each of R¹, R², R³ and R⁴ is an optionally substituted alkyl or alkylaryl group, more preferably an unsubstituted alkyl or alkylaryl group.

Any suitable anion X⁻ may be used. X⁻ may be selected from halide, acetate, nitrite, a lower alkyl sulfate, carbonate or alkyl carboxylate. Preferably X⁻ is chloride or bromide.

Each of R¹, R², R³ and R⁴ may be an unsubstituted alkyl group having from 1 to 30 carbon atoms or an alkylaryl group, for example a benzyl group.

Preferably at least one of R¹, R², R³ and R⁴ is an unsubstituted alkyl group having at least 6 carbon atoms, preferably at least 8 carbon atoms.

In one preferred embodiment R¹ is an alkyl group having from 6 to 30 carbon atoms, preferably from 8 to 24 carbon atoms, suitably from 8 to 20 carbon atoms, for example from 10 to 18 carbon atoms and most preferably from 12 to 16 carbon atoms; each of R² and R³ is an alkyl group having from 1 to 4 carbon atoms, preferably methyl and R⁴ is an alkyl group having from 1 to 4 carbon atoms, preferably methyl or an alkylaryl group, preferably benzyl. The skilled person will appreciate that such compounds may often be present as a mixture of homologues.

Suitable quaternary ammonium compounds of this type include benzyldialkyl methyl ammonium chloride and dialkyl dimethyl ammonium chloride in which the alkyl groups have 10 to 24 carbon atoms.

Some preferred quaternary ammonium compounds of this type include didecyl dimethyl ammonium chloride and dimethyl benzyl alkyl ammonium chloride in which the alkyl group contains a mixture of C₈ to C₁₆ alkyl chains.

Some suitable quaternary ammonium compounds include a substituted pyridinium compound for example an alkyl or alkenyl substituted pyridinium compound. Examples include pyridinium compounds having an alkyl or alkenyl substituent of 8 to 30, preferably 10 to 20 carbon atoms. Preferred counterions are halides. One suitable compound of this type is cetylpyridinium chloride.

Some suitable precursor compounds of this type are compounds including a guanidine moiety. The composition may comprise a compound which does not contain a permanent cation but which is protonated in solution at the pH at which the composition is used. These may be referred to as precursors to quaternary ammonium compounds. Preferred are non-polymeric guanidine compounds. Examples of such compounds include chlorhexidine salts, Chlorhexidine gluconate is especially preferred.

In some especially preferred embodiments of the method of the first aspect of the present invention the sample is contacted with a composition comprising a quaternary ammonium compound including a silicon-containing functional group. By silicon-containing group we mean to refer to any group including a silicon atom. Preferred silicon-containing functional groups are those which include a silicon atom covalently bonded via four single bonds to four organic moieties. The silicon atom may be directly bonded to oxygen and/or carbon atoms.

Preferably the method of the first aspect of the present invention component (i) comprises a compound of general formula (II): 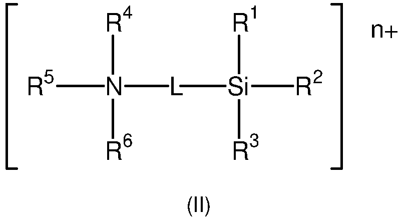 or a derivative salt thereof wherein L is a linking group; each of R¹, R², R³, R⁴, R⁵ and R⁶ is independently selected from H or an optionally substituted alkyl, alkenyl, aryl or alkoxy group; and n is 0 or 1.

It will be appreciated that in embodiments in which n is 1, the species shown in formula (I) is a cationic species.

In such embodiments the species of formula (I) will be present as an adduct or salt including a suitable counterion. However for ease of reference, in this document we may make general reference to compounds of formula (I) and any such reference includes where appropriate any counterion which must be present.

Any suitable counterion may be used. Monovalent counterions are preferred. Suitable counterions include halides and oxyhalo ions for example chloride, bromide, bromite, chlorite, hypochlorite, chlorate, bromate and iodate. In a most preferred embodiment the counterion is a chloride ion.

In this specification any optionally substituted alkyl, alkenyl, aryl or alkoxy group may be optionally substituted with one or more substituents selected from halo, hydroxy, nitro, mercapto, amino, alkyl, alkoxy, aryl, sulfo and sulfoxy.

Preferred substituents which may be present in the alkyl, alkenyl, aryl or alkoxy groups defined herein are halogens, in particular fluorine. In particular each of R¹, R², R³, R⁴, R⁵ or R⁶ may comprise fluoroalkyl or fluoroalkoxy groups which may comprise one or more fluorine atoms.

Each of R¹, R² and R³ is independently selected from an optionally substituted alkyl, alkenyl, aryl or alkoxy group. Preferably at least one of R¹, R² and R³ is an optionally substituted alkoxy group. More preferably each of R¹, R² and R³ is an optionally substituted alkoxy group, most preferably each is an unsubstituted alkoxy group. The alkyl group of the alkoxy group may be straight chained or branched. Preferably each of R¹, R² and R³ is an alkoxy group having from 1 to 20 carbon atoms, preferably from 1 to 16 carbon atoms, more preferably from 1 to 12 carbon atoms, preferably from 1 to 8 carbon atoms, suitably from 1 to 6 carbon atoms, more preferably from 1 to 4 carbon atoms.

In preferred embodiments each of R¹, R² and R³ is independently selected from methoxy, ethoxy, propoxy, butoxy and isomers thereof. Most preferably each of R¹, R² and R³ is selected from methoxy, ethoxy and isopropoxy. Preferably each of R¹, R² and R³ is selected from methoxy and ethoxy. Most preferably each of R¹, R² and R³ is methoxy. Preferably each of R¹, R² and R³ is the same.

R⁴ and R⁶ is preferably an alkyl group having from 1 to 8 carbon atoms, most preferably 1 to 6 carbon atoms, more preferably 1 to 4 carbon atoms. R⁴ and R⁶ may suitably be selected from methyl, ethyl, propyl, butyl and isomers thereof. Preferably R⁴ and R⁶ is methyl or ethyl. Most preferably R⁴ and R⁶ is methyl.

Preferably R⁵ is an alkyl group having from 8 to 30 carbon atoms, for example from 10 to 26 carbon atoms, suitably from 12 to 24 carbon atoms, preferably from 14 to 22 carbon atoms, suitably from 16 to 20 carbon atoms, for example 17 to 19 carbon atoms, suitably 18 carbon atoms.

L is a linking group. It may suitably be a bond or an optionally substituted alkylene, alkenylene or arylene group. Preferably L is an optionally substituted alkenylene group. It may be substituted along the chain or within the chain. For example L may be an ether linking moiety, i.e. a group of formula O(CH₂)ₙ in which n is 1 to 12, preferably 1 to 6.

Preferably L is an unsubstituted alkylene group, more preferably an alkylene group having 1 to 12 carbon atoms, preferably 1 to 10 carbon atoms, suitably 1 to 8 carbon atoms, for example 2 to 8 carbon atoms, more preferably 2 to 6 carbon atoms, suitably 2 to 5 carbon atoms for example 2 to 4 carbon atoms. In especially preferred embodiments L is a propylene group.

In especially preferred embodiments of the compound of formula (I), R¹, R² and R³ are each C₁ to C₄ alkoxy, L is a C₂ to C₅ alkylene group, R⁴ and R⁶ are each C₁ to C₄ alkyl groups and R⁵ is a C₁₂ to C₂₄ alkyl group.

Most preferably the compound of formula (I) is the compound shown in formula (IV). This compound is commercially available as a solution in methanol.

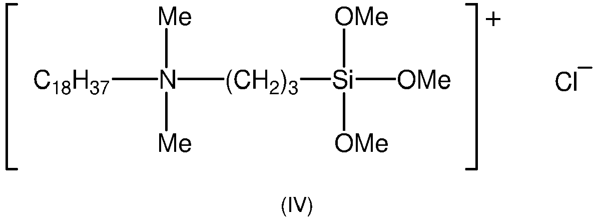

The skilled person will appreciate that commercial sources of such compounds may include some residual starting material and other minor impurities.

Preferably component (i) is selected from quaternary ammonium salts of formula (I), pyridinium salts, guanidine salts and compounds of formula (II).

More preferably component (i) comprises a compound of formula (II).

Most preferably component (i) comprises a compound of formula (IV).

In preferred embodiments the composition contacted with the sample in step (a) of the method of the present invention further comprises (ii) a non-ionic surfactant.

Component (ii) may be selected from any suitable non-ionic surfactant. Suitable non-ionic surfactants will be known to the person skilled in the art and include alcohol ethoxylates, fatty acid esters and alkyl polyglycosides.

Non-ionic surfactants may have a hydrophilic portion, suitably an alkoxylate moiety or a sugar moiety. Suitable non-ionic surfactants include alcohol ethoxylates and fatty alcohol polyglycosides. Suitably the hydrophilic-lipophilic balance (HLB) value of a non-ionic surfactant used in the present invention is at least 7, and preferably at least 10. Especially suitable non-ionic surfactants may have an HLB value falling in the range 10-16, preferably 10-14. For the purposes of these definitions HLB value is determined by the classical method of Griffin (Griffin WC: "Calculation of HLB Values of Non-Ionic Surfactants," Journal of the Society of Cosmetic Chemists 5 (1954): 249).

Preferred non-ionic surfactants for use herein include hydrocarbyl saccharide compounds. By hydrocarbyl-saccharide compound we mean to refer to a compound including a hydrocarbyl group and a saccharide moiety.

The hydrocarbyl group may be bound to the saccharide moiety via a carbon-carbon bond or via a carbon-oxygen bond. Preferably it is bound to the saccharide moiety via a carbon-oxygen bond, for example via an ester linkage or an ether linkage. Most preferably it is bound to the oligosaccharide moiety via an ether linkage. Thus in preferred embodiments the non-ionic surfactant is a hydrocarbyl ether of a saccharide moiety.

The hydrocarbyl-saccharide compound may include one or more hydrocarbyl groups. Preferably it comprises one hydrocarbyl group. The hydrocarbyl group may be an optionally substituted alkyl, alkenyl or alkynylene group. Most preferably it is an optionally substituted alkyl group. Suitable substituents include halo, hydroxy, nitro, mercapto, amino, alkyl, alkoxy, aryl, sulfo and sulfoxy. Any substitution may be within the chain or along it, for example the chain may include an ether linkage.

Preferably the hydrocarbyl group is an unsubstituted alkyl group. It may be straight chained or may be branched. Most preferably it is straight chained. Especially preferred hydrocarbyl groups are alkyl groups having from 1 to 30 carbon atoms, preferably 2 to 24 carbon atoms, more preferably from 4 to 20 carbon atoms, suitably from 4 to 16 carbon atoms, preferably from 6 to 14 carbon atoms, for example from 6 to 12 carbon atoms and most preferably from 8 to 10 carbon atoms. Preferred are straight chained alkyl groups having from 6 to 12 carbon atoms.

The saccharide moiety of the hydrocarbyl oligosaccharide species may include from 1 to 10 monosaccharide species. Thus it may be a monosaccharide unit, a disaccharide unit or an oligosaccharide unit. Preferably the saccharide moiety comprises from 2 to 8, suitably from 2 to 6, preferably from 2 to 5, for example 3 or 4 monosaccharide units. Any suitable monosaccharide unit may be included. Preferred saccharides include allose, altrose, glucose, mannose, gulose, idose, galactose and talose.

Mixtures of two or more monosaccharides may be present in the saccharide moiety. Preferably the saccharide moiety comprises glucose. More preferably all of the monosaccharide units present in the saccharide moiety are glucose.

In a preferred embodiment the non-ionic surfactant is an alkyl polyglucoside (APG), preferably a monoalkyl-polyglucoside. Suitably the non-ionic surfactant is a compound of general formula (III): 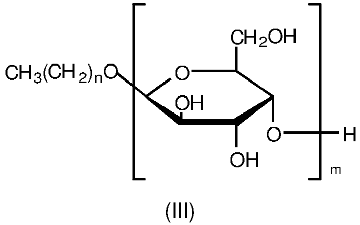 wherein n is from 5 to 12, preferably from 6 to 10, more preferably from 7 to 9 and m is from 1 to 6, preferably from 2 to 5, more preferably 3 or 4.

The composition contacted with the sample in step (a) of the method of the first aspect may be provided in any suitable form. It may consist essentially of component (i) or component (i) and a solvent. It may consist essentially of components (i) and (ii) or it may comprise one or more further components. Suitably the composition includes one or more solvents. Preferred solvents are water and water miscible solvents. In embodiments in which the quaternary ammonium compound is obtained commercially as a solution in methanol, much of the methanol is suitably removed prior to use of the compound in the method of the present invention.

Preferably the composition is aqueous. In especially preferred embodiments water comprises at least 90 wt%, more preferably at least 95 wt% or at least 99 wt% of all solvents present in the composition. In one embodiment the composition is freeze dried. In such embodiments an aqueous mixture may be provided upon contact with the sample. Freeze-dried compositions may be advantageous for storage and distribution.

In some embodiments the composition used in step (a) may be immobilised on a solid support, for example on a resin bead or on a planar substrate.

The composition used in step (a) of the method of the present invention may include a mixture of two or more quaternary ammonium compounds and/or a mixture of two or more non-ionic surfactants.

The composition contacted with the sample in step (a) of the method of the present invention preferably comprises at least 0.0001 wt% of a quaternary ammonium compound, preferably at least 0.0005 wt%, more preferably at least 0.001 wt%, and more preferably at least 0.002 wt%.

The quaternary ammonium compound preferably comprises up to 10 wt% of the composition contacted with the sample in step (a), suitably up to 5 wt%, preferably up to 1 wt%, preferably up to 0.1 wt%, more preferably up to 0.01 wt%, and more preferably up to 0.005 wt%.

In embodiments in which more than one quaternary ammonium compound is present, the above amounts refer to the total of all such compounds.

The non-ionic surfactant is suitably present in the composition contacted with the sample in step (a) in an amount of at least 0.0001 wt%, preferably at least 0.0005 wt%, more preferably at least 0.001 wt%, and more preferably at least 0.002 wt%.

The non-ionic surfactant may be present in the composition contacted with the sample in step (a) in an amount of up to 10 wt% of the composition, suitably up to 5 wt%, preferably up to 1 wt%, preferably up to 0.1 wt%, more preferably up to 0.01 wt%, and more preferably up to 0.005 wt%.

In embodiments in which the composition comprises two or more non-ionic surfactants the above amounts refer to the total of all such compounds.

The weight ratio of the quaternary ammonium compound functional component to the non-ionic surfactant is preferably from 1:10 to 10:1, preferably from 1:5 to 5:1, preferably from 1:3 to 3:1, suitably from 1:2.5 to 2.5:1.

The composition contacted with the sample in step (a) preferably has a pH of from 6 to 8.
The composition contacted with the sample preferably comprises (i) a quaternary ammonium compound and (ii) a non-ionic surfactant. In some embodiments the composition may consist essentially of these components.

In some embodiments the composition further includes a solvent, preferably water.

Other components may also be present, for example magnesium chloride and tris buffers. However this is not preferred.

Suitably components (i) and (ii), which may each comprise a mixture of components, together make up at least 50 wt% of all ingredients other than solvent present in the composition used in step (a), preferably at least 70 wt%, more preferably at least 90 wt%, preferably at least 95 wt%, for example at least 99 wt%.

Preferably the composition used in step (a) of the method of the present invention comprises less than 0.01 mmol of magnesium ions, preferably less than 0.001 mmol.

In step (a) of the method of the first aspect of the present invention the sample is contacted with a composition comprising (i) a quaternary ammonium compound and optionally (ii) a non-ionic surfactant. Suitably the ratio of the sample composition (e.g. blood) to the composition used in step (a) is from 10:1 to 1:1000, preferably from 5:1 to 1:100, suitably from 1:1 to 1:20 for example from 1:3 to 1:10, by volume.

Suitably the composition is agitated to ensure mixing.

The present invention provides a method of stabilising genetic material. By stabilising the genetic material we mean that degradation or decomposition of the genetic material is reduced, inhibited, prevented or delayed.

For example the present inventors have surprisingly found that genetic material treated according to the method of the present invention retains its integrity to a greater extent and over longer periods compared to genetic material that is not treated according to the invention.

Thus the present invention may enable genetic material to be stored. Suitably the stored material does not significantly degrade or decompose during the storage time.

Suitably genetic material treated according to the present disclosure has a storage stability of at least 50% when stored under ambient conditions for 1 hour. Preferably genetic material treated according to the present invention has a storage stability of at least 70%, preferably at least 80%, more preferably at least 90%, suitably at least 90%, for example at least 100% when stored under ambient conditions for 1 hour.

By a storage stability of at least 50% we mean that at least 50% of the genetic material maintains its integrity during the storage period. Thus analysis of the genetic material would show at least 50% identity before and after the storage period.

Suitably genetic material treated according to the present disclosure has a storage stability of at least 50% when stored under ambient conditions for 3 hours. Preferably genetic material treated according to the present invention has a storage stability of at least 70%, preferably at least 80%, more preferably at least 90%, suitably at least 90%, for example at least 100% when stored under ambient conditions for 3 hours.

Suitably genetic material treated according to the present invention has a storage stability of at least 50% when stored under ambient conditions for 24 hours. Preferably genetic material treated according to the present invention has a storage stability of at least 70%, preferably at least 80%, more preferably at least 90%, suitably at least 90%, for example at least 100% when stored under ambient conditions for 24 hours.

Suitably genetic material treated according to the present invention has a storage stability of at least 50% when stored under ambient conditions for 1 week. Preferably genetic material treated according to the present invention has a storage stability of at least 70%, preferably at least 80%, more preferably at least 90%, suitably at least 90%, for example at least 100% when stored under ambient conditions for 1 week.

Suitably genetic material treated according to the present invention has a storage stability of at least 50% when stored under ambient conditions for 2 weeks. Preferably genetic material treated according to the present invention has a storage stability of at least 70%, preferably at least 80%, more preferably at least 90%, suitably at least 90%, for example at least 100% when stored under ambient conditions for 2 weeks.

Suitably genetic material treated according to the present invention has a storage stability of at least 50% when stored under ambient conditions for 1 month. Preferably genetic material treated according to the present invention has a storage stability of at least 70%, preferably at least 80%, more preferably at least 90%, suitably at least 90%, for example at least 100% when stored under ambient conditions for 1 month.

Stabilisation of genetic material according to the present invention may delay the degradation of the genetic material and/or may reduce the extent of the degradation of the genetic material.

The method of the present invention provides stabilisation of the genetic material. This facilitates storing and/or transporting the material.

The method of the present disclosure may involve storing the genetic material for at least 30 minutes, suitably at least 1 hour. The method of the present invention may involve storing the genetic material more than 6 hours, suitably more than 12 hours, for example more than 24 hours or more than 48 hours.

In some embodiments the genetic material may be stored for more than 3 days, for example more than one week or more than one month.

In the present invention the composition obtained may be stored under ambient conditions. Typically ambient conditions involve atmospheric pressure and temperatures between 5°C and 40°C. The composition is suitably stored in a sealed container but preferably no special conditions are needed. Without being bound by theory it is believed that the method of the present invention causes lysis of cells present in the sample. Genetic material is thus released from the cells. However it is stabilised by interaction with the composition comprising the quaternary ammonium salt and does not break down as is often the case, particularly with RNA, following cell lysis.

A particular advantage of the present invention is that genetic material remains stable even in the presence of RNAse. RNAse is naturally present in most biological samples and is one reason why RNA in particular is not usually storage stable and readily degrades unless it is purified. Thus stabilisation of genetic material, especially RNA is a significant advantage of the present invention.

A further advantage of the present invention is that it enables stabilisation of all of the genetic material present in a sample. Suitably the invention lyses the cells in a sample and stabilises the genetic material released.

It is an advantage of the present invention that the stabilised genetic material may be stored such that it can be used in the fields of, for example, forensics and diagnostics. It is a particular advantage that, for example, a sample may be taken at one location, the genetic material may be stabilised immediately and then be stored and transported to a different location for analysis.

Accordingly, the present invention may optionally comprise a step of detection of the presence or absence of target genetic material, suitably target DNA and/or RNA.

Detection of the presence or absence of target genetic material, suitably target DNA and/or RNA, involves examining the genetic material present in the one or more samples.

Techniques for examining DNA and/or RNA are known to the person skilled in the art and may involve any suitable method.

It is an advantage of the present invention that because the genetic content of a whole cell or groups of cells can be stabilised a genetic fingerprint of the cell or cells can be determined at multiple time points. For example a sample may be taken from a patient at a first time point (T1) and a second time point (T2). The genetic material in the samples taken at both T1 and T2 may be stabilised using the method of the present invention and the samples may be subsequently analysed and compared.

It is an advantage of the present invention that the genetic material present in the whole sample may be stabilised and subsequently analysed.

The present invention may find utility in a variety of applications, particularly in the fields of diagnosis and forensics.

According to a second aspect of the present invention there is provided a method of analysing genetic material in a sample, the method comprising the steps of:
(a) treating the one or more samples according to the method of the first aspect;
(b) optionally storing and/or transporting one or more samples;
(c) optionally contacting the composition obtained in step (a) with a composition comprising a proteinceous washing agent; and
(d) examining the genetic material present in one or more samples.

The method of the second aspect of the present invention mat be carried out on one or more samples. Where appropriate features defined in relation to one sample may apply equally to other samples.

Step (b) involves storing and/or transporting the one or more samples. Thus the present invention may be using to analyse samples collected at multiple or remote locations.

A particular advantage of the present invention is that samples can be stored at ambient temperature without degrading. This means that multiple samples can be collected, transported if necessary and stacked. Analysis can then be carried out on a batch of samples which is usually much more efficient.

In some embodiments the method of the second aspect of the present invention may optionally comprise a step (c) of contacting the composition obtained in step (a) with a composition comprising a proteinaceous washing agent.

Suitably there are no purification steps between step (a) and step (c) and the material obtained in step (a) is used directly in step (c), optionally after storing and/or transporting according to step (b).

Preferred proteinaceous washing agents are anionic proteins.

Suitable proteinaceous washing agents include tryptone, gelatin, casein and bovine serum albumin (BSA). Preferred proteinaceous washing agents include bovine serum albumin and casein. An especially preferred washing agent is BSA. Acetylated bovine serum albumin (BSA) is particularly preferred.

Suitably the proteinaceous washing agent is present in the composition used in step (c) in an amount from 0.01 to 50 wt%, preferably 0.1 to 10 wt%, suitably from 0.1 to 5 wt%, for example about 1 wt%.

Suitably the composition used in step (c) of the method of the second aspect of the present invention is an aqueous composition. Suitably water comprises at least 90 wt% of all solvents present in the composition, preferably at least 95 wt% for example at least 99 wt% of all solvents present in the composition.

In step (c) of the method of the second aspect of the present invention suitably the composition obtained in step (a) is added to a composition containing the proteinaceous washing agent in a ratio of from 10:1 to 1:100, preferably from 5:1 to 1:50, suitably from 1:1 to 1:10.

In one embodiment the composition may be freeze dried. In such an embodiment an aqueous mixture may be provided upon contact with the aqueous composition obtained in step (a).

Suitably in step (c) of the method of the present invention the mixture is briefly agitated at room temperature. It may be left for a period of 1 second to 24 hours, suitably 5 seconds to 1 hour, preferably 5 seconds to 30 minutes, preferably 10 seconds to 10 minutes, suitably from 30 seconds to 5 minutes, for example about 1 minute.

It is advantageous to include step (c), contacting the composition obtained in step (a) with a proteinaceous washing agent, in embodiments in which step (d) involves amplifying genetic material.

In some embodiments step (d) may involve detecting the presence or absence of target genetic material, such as target DNA and/or RNA, using hybridisation methods, melt analysis methods, amplification methods or sequencing methods.

Suitable hybridisation methods involve the use of hybridisation probes. Suitable hybridisation probes include single stranded DNA probes, double stranded DNA probes and RNA probes such as cRNA probes or riboprobes. The hybridisation probes may be tagged with radioactive, fluorescent or chemiluminescent molecules, suitably fluorescent molecules.

Suitable melt analysis methods include high resolution melt (HRM) analysis. Suitably melt analysis methods involve the use of dyes. Suitable dyes include fluorescent and chemiluminescent dyes, suitably fluorescent dyes.

Suitable amplification methods include polymerase chain reaction (PCR), isothermal amplification, loop-mediated isothermal amplification (LAMP), strand displacement amplification (SDA), helicase-dependent amplification (HDA) and nicking enzyme amplification reaction. Suitable PCR methods include basic PCR, reverse transcription PCR (RT-PCR), endpoint reverse transcription PCR, hot-start PCR, long PCR, quantitative PCR (qPCR), quantitative endpoint PCR, quantitative reverse transcription (qRT-PCR), rapid amplified polymorphic DNA analysis, nested PCR and high-fidelity PCR.

Suitable sequencing methods include Maxam-Gilbert sequencing, chain-termination sequencing (also known as Sanger sequencing), shotgun sequencing, bridge PCR (polymerase chain reaction), single-molecule real time sequencing (SMRT), ion semiconductor sequencing, pyrosequencing, sequencing by synthesis (illumina sequencing), sequencing by ligation (SOLiD), massively parallel signature sequencing (MPSS), polony sequencing, 454 sequencing, DNA nanoball sequencing, heliscope single molecule sequencing, nanopore DNA sequencing, tunnelling currents DNA sequencing, sequencing by hybridisation, sequencing with mass spectrometry, microfluidic Sanger sequencing, RNAP sequencing and microscopy-based sequencing.

In some embodiments step (d) may involve contacting the sample with a probe which selectively binds to a specific DNA and/or RNA sequence. The presence or absence of a signal (e.g. fluorescence) which occurs on binding indicates the presence or absence of the specific DNA and/or RNA sequence.

In such embodiments the inclusion of step (c) may or may not be necessary.

In some embodiments step (d) may involve amplifying one or more components of genetic material present in each sample. Suitable amplification methods are known to the person skilled in the art and preferred methods are based on PCR techniques.

Suitably in embodiments in which step (d) involves a PCR method added to the sample which are known to bind to a target DNA and/or RNA molecule.

In embodiments in which step (d) involves an amplification method, for example PCR, the method of the second aspect preferably includes step (c). In preferred embodiments step (c) is carried out directly on the material obtained in step (a) without any washing or purification steps.

A particular advantage of embodiments of the present invention is that a specific RNA target sequence can be identified. Many methods of the prior art are unable to detect RNA due to its poor stability.

Detection of RNA is highly advantageous particularly in the field of diagnostics because it is present in greater concentrations than DNA, thus allowing lower limits of detection to be achieved. Furthermore RNA provides more up to date information regarding the current activity of a cell.

In some alternative embodiments step (d) of the method of the present disclosure may involve recording the genetic fingerprint of a first and a second sample and comparing these. In such embodiments it is not necessary to determine absolutely all or indeed any of the particular components of genetic material. Rather such a method can enable the comparison of two samples to establish the similarities or differences. In the field of forensics this could enable a determination of whether two or more samples are taken from the sample source.

In diagnosis or disease monitoring samples from the same patient can be taken over time to monitor how a disease is progressing. This can also help with diagnosis.

Although it is not necessary, in some embodiments when comparisons are made to determine absolutely the identity of specific components, this can be beneficial and provide extra information, for example, to assist with a diagnosis.

In some embodiments the method of the second aspect may be used in the detection or diagnosis of a disease. The method may be used to detect or diagnose, among others, genetic diseases, cancers, autoimmune diseases and pathogenic diseases. Genetic markers indicative of disease are identified in step (d). One particular advantage of the present invention is that because samples do not need to be purified before or after step (d) there is significant reduction in the time taken to reach diagnosis. As mentioned above, lower limits of detection may also be achieved. Furthermore samples may be collected at a first location and safely transported to a second location for analysis without degradation of the genetic material.

In some embodiments the method of the second aspect may be used to detect or diagnose infection with a pathogenic disease. Suitably the method of the second aspect of the present invention may be used to detect or diagnose infection with a pathogenic disease is which a target DNA and/or RNA sequence characteristic of the pathogenic microorganism can be identified.

Suitable pathogenic organisms include pathogenic bacteria of the genera *Bacteroides, Bartonella, Bordetella, Borrelia, Brucella, Campylobacter, Chlamydia, Escherichia, Francisella, Haemophilus, Helicobacter, Klebsiella, Legionella, Leptospira, Moraxella, Neisseria, Pasteurella, Proteus, Pseudomonas, Salmonella, Shigella, Spirillum, Streptobacillus, Treponema, Vibro, Yersinia, Actinomyces, Bacillus, Clostridium, Corynebacterium, Listeria, Nocardia, Peptostreptococcus, Propionibacterium, Staphylococcus, Streptococcus* and *Streptomyces,* pathogenic protozoa of the genera *Acanthamoeba, Ancylostoma, Ascaradia, Babesia, Balamuthia, Balantidium, Brugia, Clonorchis, Cryptosporidium, Dicrocoelium, Dicytocaulus, Dientamoeba, Diphylobothrium, Dirofilaria, Echinococcus, Echinostoma, Entamoeba, Enterobius, Fasciola, Fascioloides, Giardia, Hymenolepsis, Isospora, Leishmania, Mesocestoides, Moniezia, Necator, Naegleria, Onchocerca, Opisthorchis, Paragonimus, Plasmodium, Rhabditida, Schistosoma, Spirurida, Strongyloides, Taenia, Trichomonas, Trichuris, Toxocara, Trypanosoma, Uncinaria* and *Wuchereria.*

In some embodiments method of the second aspect of the may be used to detect or diagnose cancers.

Suitable cancers include cancers derived from brain cells, epithelial cells (carcinoma), connective tissue (sarcoma), hematopoietic cells (lymphoma and leukemia), pluripotent cells (germ cell tumour), and embryonic tissue (blastoma). Suitable cancers include, among others, breast cancer, brain cancer, liver cancer, stomach cancer, skin cancer, prostate cancer, cervical cancer, lung cancer.

An advantage of the present invention is that because the entire DNA and/or RNA content of a cell or group of cells can be stabilised a genetic fingerprint can be determined at multiple points during the progression of a disease. This is particularly in some cases, for example breast cancer, where genetic mutations accumulate as the disease progresses.

In some embodiments the method of the present disclosure may be used to detect or diagnose autoimmune diseases. Autoimmune diseases commonly affect organ and tissue types such as blood vessels, connective tissues, endocrine glands, joints, muscles, red blood cells, and the skin.

Suitable example of autoimmune diseases include, among others, Addison's disease, celiac disease, dermatomyositis, Graves' disease, Guillan-Barre disease, inflammatory bowel disease, multiple sclerosis, pernicious anaemia, psoriasis, rheumatoid arthritis, systemic lupus erythematosus and type I diabetes.

The present invention may have particular utility in the field of personalised medicine where specific mutations in, for example, a cancer can be used to prescribe particular drugs.

The method of the present invention may have particular utility in the field of companion diagnostics.

The method of the present invention may have particular utility in the forensics.

According to a third aspect of the present invention there is provided the use of a quaternary ammonium compound as defined in claim 9 to stabilise genetic material.

Preferred features of the third aspect are as defined in relation to the first and second aspects as appropriate.

The invention will now be further defined with reference to the following non-limiting examples.

### Example 1

Composition A was prepared comprising, in an aqueous solution:
- 0.003 wt% of a quaternary ammonium compound having the formula: 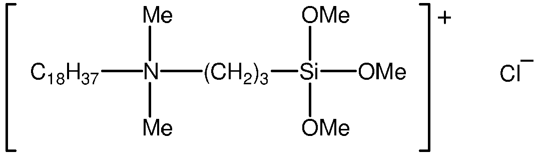
- 0.003 wt% of an alkyl polyglucoside alkyl polyglucoside comprising a mixture of isomers of formula: 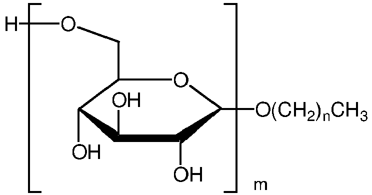 where n = 7 or 9 and m = 1 to 5; and

Composition B was prepared comprising water and 1 wt% acetylated bovine serum albumin (BSA).

### Example 2

One blood sample was collected using an ISO-compliant tube for diagnostic containing EDTA. The fresh sample (350 µL) was extracted according to the RNA-pure system in the Qiagen (RTM) EZ-1 automated and self-contained robot, following the manufacturer's standard protocol and obtaining 50 µl of concentrated extract. 5 µl of this sample was used in a Qiagen standard qRT-PCR giving a count of 25.75. The blood sample was then stored at -20°C.

### Sample 2A

30 µL of defrosted whole blood were mixed with 3 µL of composition A and 167 µL of water (molecular biology grade). The mix was vortexed and incubated at room temperature 1 minute.

2.5 µL of sample 2A was mixed with 10 µL of composition B. A RT-qPCR for *Plasmodium* spp. was then performed in the StepOne Real-Time PCR System (Applied Biosystems) with the following thermal profile:

| | Temperature | Time | No. cycles |
|---|---|---|---|
| Initial step for reverse transcription | 45 °C | 10 min | 1 cycle |
| Initial denaturation | 95 °C | 10 min | 1 cycle |
| Denaturation | 95 °C | 15 sec | 45 cycles |
| Annealing | 60 °C | 60 sec | |

The following mastermixes were used:

**Mastermix 1 (reverse transcription (RT) mix boosted with RT enzyme mix)**

| Component | Amount |
|---|---|
| AgPath-ID™ One-Step RT-PCR 25x enzyme mix | |
| NZY qPCR 2x mastermix (Nzytech) | |
| 0.1 µM forward primer | |
| (5'-GTTAAGGGAGTGAAGACGATCAGA-3' (SEQ ID NO. 1)) | |
| 0.1 µM reverse primer | |
| (5'AACCCAAAGACTTTGATTTCTCATAA-3' (SEQ ID NO.2)) | 15 µl |
| 0.05 µM forward control primer (hRnaseP) | |
| 0.05 µM reverse control primer (hRnaseP) | |
| 0.1 µM *Plasmodium* probe | |
| (5'-FAM-ACCGTCGTAATCTTAACCATAAACTATGCCGACTAG-TAMRA-3' (SEQ ID NO.3)) | |
| 0.02 µM internal control probe | |

**Mastermix 2 (standard reverse transcription (RT) mix)**

| Component | Amount |
|---|---|
| AgPath-ID™ One-Step RT-PCR 25x enzyme mix and Buffer RT | 15 µl |
| 0.1 µM forward primer | |
| (5'-GTTAAGGGAGTGAAGACGATCAGA-3' (SEQ ID NO. 1)) | |
| 0.1 µM reverse primer | |
| (5'AACCCAAAGACTTTGATTTCTCATAA-3' (SEQ ID NO.2)) | |
| 0.05 µM forward control primer (hRnaseP) | |
| 0.05 µM reverse control primer (hRnaseP) | |
| 0.1 µM *Plasmodium* probe | |
| (5'-FAM-ACCGTCGTAATCTTAACCATAAACTATGCCGACTAG-TAMRA-3' (SEQ ID NO.3)) | |
| 0.02 µM internal control probe | |

Mastermix 1 amplifies both DNA and RNA present in the sample; mastermix 2 amplifies only RNA.

The results of the PCR (counts) are shown in table 1:

**Table 1**

| | Mastermix 1 | Mastermix 2 |
|---|---|---|
| Sample 2A (inv) | 17.43 | 19.64 |

The results show that detection of RNA and DNA according to the present invention allows lower levels of plasmodium to be detected compared to the Qiagen standard procedure. It should also be noted that the method of the present invention uses only 30 µL of the blood whereas the Qiagen method uses 350 µL. This represents a substantial benefit, for example in diagnosis as many more tests (eg for different diseases) can be carried out on the same amount of blood.

Sample 2A was split and stored at either room temperature for 26 days (624h) or -20°C for 6 days (144h). The PCR experiments were repeated after 24h, 48h, 144h and 624h. The results are shown in table 2.

| | Mastermix 1 | Mastermix 2 |
|---|---|---|
| 24h, room temp | 17.38 | 19.69 |
| 24h, -20°C | 17.66 | 19.46 |
| 48h, room temp | 18.25 | 21.4 |
| 48h, -20°C | 17.74 | 20.61 |
| 144h, room temp | 18.76 | 21.36 |
| 144h, -20°C | 19.69 | 22.31 |
| 624h, room temp | 19.35 | 22.67 |

These results show how the method of the present invention still allows RNA and DNA to be detected following storage.

### Example 3

Buccal swaps are important in forensic applications. These are typically collected in the field, stored and batched until processing.

In this experiment 4 buccal swabs were taken from the same donor, wetted with PCR grade water and dropped into a sample tube containing composition A. The samples were stored at ambient temperature for 3 weeks. Each week the stored samples were tested, with hRNaseP qPCR, to determine if the sample was still amplifiable.

**Master Mix**

| **hRNaseP** | **1x** | **10X** |
|---|---|---|
| MasterMix | 12.5 | 125 |
| Forward primer (50µM) | 0.4 | 4 |
| AGATTTGGACCTGCGAGCG (SEQ ID NO. 4) | | |
| R primer (50µM) | 0.4 | 4 |
| GAGCGGCTGTCTCCACAAGT (SEQ ID NO. 5) | | |
| Probe (1µM) | 0.5 | 5 |
| VIC-TTCTGACCTGAAGGCTCTGCGCG-TAMRA (SEQ ID NO. 6) | | |
| Water | 6.2 | 62 |

**Results (Ct values for hRNAseP assay)**

| Fresh Swab | 24h stored | 1 Week stored | 2 Weeks Stored | 3 Weeks Stored |
|---|---|---|---|---|
| 36.04 | 33.3 | 32.24 | 30.51 | 30.21 |
| 35.09 | 33.62 | 32.24 | 30.42 | 30.44 |

## Claims

1. A method of stabilising DNA and/or RNA in a sample, the method comprising steps of:
(a) contacting the sample with a composition comprising
(i) a quaternary ammonium compound including a silicon-containing functional group of formula: 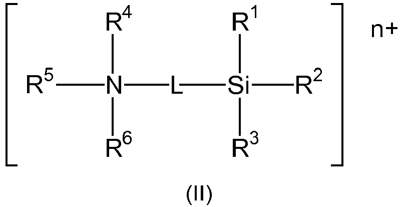 or a derivative salt thereof wherein L is a linking group; each of R¹, R², R³, R⁴, R⁵ and R⁶ is independently selected from H or an optionally substituted alkyl, alkenyl, aryl or alkoxy group; and n is 0 or 1;
wherein the method provides a storage stability of at least 50% when the sample is stored under ambient conditions for 24 hours.

2. A method according to claim 1 wherein the sample is a sample of bodily fluid or tissue obtained from a human.

3. A method according to any preceding claim wherein the composition contacted with the sample in step (a) of the method of the present invention further comprises (ii) a non-ionic surfactant.

4. A method according to claim 4 wherein the the non-ionic surfactant is an alkyl polyglucoside.

5. A method of analysing DNA and/or RNA in a sample, the method comprising the steps of:
(a) treating the one or more samples according to the method of any preceding claim;
(b) optionally storing and/or transporting one or more samples;
(c) optionally contacting the composition obtained in step (a) with a composition comprising a proteinaceous washing agent; and
(d) examining the DNA and/or RNA present in one or more samples.

6. A method according to claim 5 wherein there are no purification steps between step (a) and step (c).

7. A method according to claim 5 or claim 6 wherein the proteinaceous washing agent is selected from bovine serum albumin and casein.

8. A method according to any of claims 5 to 7 wherein step (d) involves detecting the presence or absence of target DNA and/or RNA, using hybridisation methods, melt analysis methods, amplification methods or sequencing methods.

9. Use of a quaternary ammonium compound including a silicon-containing functional group of formula: 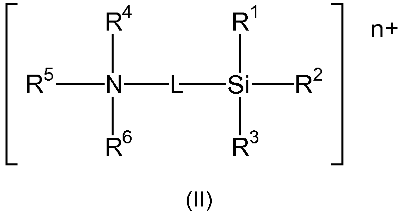 or a derivative salt thereof wherein L is a linking group; each of R¹, R², R³, R⁴, R⁵ and R⁶ is independently selected from H or an optionally substituted alkyl, alkenyl, aryl or alkoxy group; and n is 0 or 1 to stabilise DNA and/or RNA.

## Patentansprüche

1. Verfahren zur Stabilisierung von DNA und/oder RNA in einer Probe, wobei das Verfahren folgende Schritte umfasst:
(a) Inkontaktbringen der Probe mit einer Zusammensetzung, umfassend
(i) eine quartäre Ammoniumverbindung mit einer siliciumhaltigen funktionellen Gruppe der Formel: 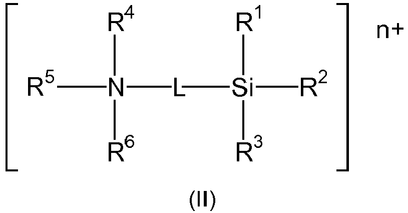 oder ein Derivatsalz davon, wobei L für eine Verknüpfungsgruppe steht, R¹, R², R³, R⁴, R⁵ und R⁶ jeweils unabhängig aus H oder einer gegebenenfalls substituierten Alkyl-, Alkenyl-, Aryl- oder Alkoxygruppe ausgewählt sind und n für 0 oder 1 steht;
wobei das Verfahren eine Lagerstabilität von mindestens 50 % bei Lagerung der Probe unter Umgebungsbedingungen über einen Zeitraum von 24 Stunden bereitstellt.

2. Verfahren nach Anspruch 1, wobei es sich bei der Probe um eine von einem Menschen erhalten Probe von Körperflüssigkeit oder Körpergewebe handelt.

3. Verfahren nach einem der vorhergehenden Ansprüche, wobei die in Schritt (a) des Verfahrens der vorliegenden Erfindung mit der Probe in Kontakt gebrachte Zusammensetzung ferner (ii) ein nichtionisches Tensid umfasst.

4. Verfahren nach Anspruch 4, wobei es sich bei dem nichtionischen Tensid um ein Alkylpolyglukosid handelt.

5. Verfahren zur Analyse von DNA und/oder RNA in einer Probe, wobei das Verfahren folgende Schritte umfasst:
(a) Behandeln der Probe bzw. der Proben gemäß dem Verfahren nach einem der vorhergehenden Ansprüche;
(b) gegebenenfalls Lagern und/oder Transportieren einer oder mehrerer Proben;
(c) gegebenenfalls Inkontaktbringen der in Schritt (a) erhaltenen Zusammensetzung mit einer Zusammensetzung, die ein proteinartiges Waschmittel umfasst; und
(d) Untersuchen der DNA und/oder RNA in einer oder mehreren Proben.

6. Verfahren nach Anspruch 5, bei dem zwischen Schritt (a) und Schritt (c) keine Reinigungsschritte durchgeführt werden.

7. Verfahren nach Anspruch 5 oder Anspruch 6, wobei das proteinartige Waschmittel aus Rinderserumalbumin und Casein ausgewählt ist.

8. Verfahren nach einem der Ansprüche 5 bis 7, wobei in Schritt (d) die Anwesenheit oder Abwesenheit von Ziel-DNA und/oder -RNA unter Verwendung von Hybridisierungsverfahren, Schmelzanalyseverfahren, Amplifikationsverfahren oder Sequenzierungsverfahren nachgewiesen wird.

9. Verwendung einer quartären Ammoniumverbindung mit einer siliciumhaltigen funktionellen Gruppe der Formel: 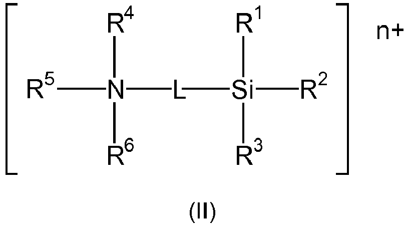 oder eines Derivatsalzes davon, wobei L für eine Verknüpfungsgruppe steht, R¹, R², R³, R⁴, R⁵ und R⁶ jeweils unabhängig aus H oder einer gegebenenfalls substituierten Alkyl-, Alkenyl-, Aryl- oder Alkoxygruppe ausgewählt sind und n für 0 oder 1 steht, zur Stabilisierung von DNA und/oder RNA.

## Revendications

1. Méthode destinée à stabiliser de l'ADN et/ou de l'ARN dans un échantillon, la méthode comprenant les étapes consistant à :
(a) mettre l'échantillon en contact avec une composition comprenant
(i) un composé d'ammonium quaternaire comportant un groupement fonctionnel contenant du silicium, de formule : 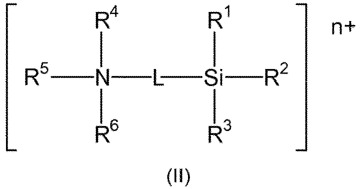 ou un sel dérivé de celui-ci, où L est un groupement de liaison ; chacun parmi R¹, R², R³, R⁴, R⁵ et R⁶ est choisi indépendamment parmi H ou un groupement alkyle, alcényle, aryle ou alcoxy éventuellement substitué ; et
n vaut 0 ou 1 ;
la méthode fournissant une stabilité au stockage d'au moins 50% lorsque l'échantillon est stocké dans des conditions ambiantes pendant 24 heures.

2. Méthode selon la revendication 1, dans laquelle l'échantillon est un échantillon de fluide ou de tissu corporel obtenu auprès d'un être humain.

3. Méthode selon l'une quelconque des revendications précédentes, dans laquelle la composition mise en contact avec l'échantillon dans l'étape (a) de la méthode de la présente invention comprend en outre (ii) un agent tensioactif non ionique.

4. Méthode selon la revendication 4, dans laquelle l'agent tensioactif non ionique est un polyglucoside d'alkyle.

5. Méthode destinée à l'analyse d'ADN et/ou d'ARN dans un échantillon, la méthode comprenant les étapes consistant à :
(a) traiter les un ou plusieurs échantillons selon la méthode selon l'une quelconque des revendications précédentes ;
(b) éventuellement stocker et/ou transporter les un ou plusieurs échantillons ;
(c) éventuellement mettre en contact la composition obtenue dans l'étape (a) avec une composition comprenant un agent de lavage protéique ; et
(d) examiner l'ADN et/ou l'ARN présents dans les un ou plusieurs échantillons.

6. Méthode selon la revendication 5, dans laquelle il n'y a aucune étape de purification entre l'étape (a) et l'étape (c).

7. Méthode selon la revendication 5 ou la revendication 6, dans laquelle l'agent de lavage protéique est choisi parmi la sérum albumine bovine et la caséine.

8. Méthode selon l'une quelconque des revendications 5 à 7, dans laquelle l'étape (d) met en jeu la détection de la présence ou de l'absence d'ADN et/ou d'ARN cible, en utilisant des méthodes d'hybridation, des méthodes d'analyse à l'état fondu, des méthodes d'amplification ou des méthodes de séquençage.

9. Utilisation d'un composé d'ammonium quaternaire comportant un groupement fonctionnel contenant du silicium, de formule : 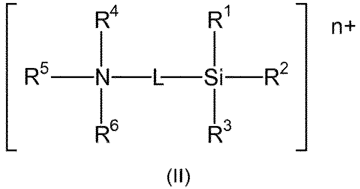 ou d'un sel dérivé de celui-ci, où L est un groupement de liaison ; chacun parmi R¹, R², R³, R⁴, R⁵ et R⁶ est choisi indépendamment parmi H ou un groupement alkyle, alcényle, aryle ou alcoxy éventuellement substitué ; et n vaut 0 ou 1 ; afin de stabiliser de l'ADN et/ou de l'ARN.
